# EUROPÄISCHE PATENTANMELDUNG

(11) **EP 2 489 778 A2**
(43) Veröffentlichungstag der Anmeldung: **22.08.2012**
(21) Anmeldenummer: 12154376.3
(22) Anmeldetag: 08.02.2012
(51) Int. Cl.: D06L 3/02, D06L 3/16

(54) **Hygiene- und/oder Kosmetikprodukt, Vliesstoff und Fasern hierfür, sowie Behandlungsverfahren für Kapokfasern**

(30) Priorität: 18.02.2011 DE 102011004377
(71) Anmelder: Gebrüder Otto Baumwollfeinzwirnerei GmbH & Co. KG, 89165 Dietenheim (DE)
(72) Erfinder: Merkel, Andreas, 89165 Dietenheim (DE); Kächler, Oliver, 88481 Balzheim (DE)
(74) Vertreter: Bergmeier, Werner

(57) **Zusammenfassung**

Vorgeschlagen wird ein Behandlungsverfahren für Kapokfasern (5), bei dem eine natürliche Zytotoxizität der Kapokfasern (5) beseitigt wird, so dass die Kapokfasern für Hygiene- und/oder Kosmetikprodukte verwendbar sind.

Ebenso vorgeschlagen werden Kapokfasern (5), ein Vliesstoff (2, 3, 4) sowie ein Hygiene- und/oder Kosmetikprodukt (1).

## Beschreibung

Die vorliegende Erfindung betrifft ein Behandlungsverfahren für Kapokfasern für Hygiene- und/oder Kosmetikprodukte. Ebenso betrifft die Erfindung Kapokfasern und einen Vliesstoff für Hygiene- und/oder Kosmetikprodukte. Darüber hinaus betrifft die Erfindung ein Hygiene- und/oder Kosmetikprodukt, insbesondere ein Wattepad, eine Stilleinlage, einen Tampon, ein Wattestäbchen, ein Wundversorgungsprodukt oder ein Inkontinenzprodukt, umfassend wenigstens einen Vliesstoff.

Es ist bekannt, Hygiene- und/oder Kosmetikprodukte, insbesondere Wattepads, Stilleinlagen, Tampons, Wattestäbchen, Wundversorgungsprodukte oder Inkontinenzprodukte, aus Vliesstoffen herzustellen, welche ihrerseits unter Verwendung von Fasern hergestellt werden.

Aufgabe der vorliegenden Erfindung ist es, Hygiene- und/oder Kosmetikprodukte der genannten Art zu verbessern.

Die Aufgabe wird bei einem Behandlungsverfahren der eingangs genannten Art dadurch gelöst, dass eine natürliche Zytotoxizität der Kapokfasern beseitigt wird, so dass die Kapokfasern für Hygiene- und/oder Kosmetikprodukte verwendbar sind.

Bei einer Kapokfaser, auch Pflanzendaune genannt, handelt es sich um eine natürliche Hohlfaser, die vorwiegend vom Kapokbaum (wiss.: Ceiba Pentandra), aber auch von einigen Wollbaumgewächsen gewonnen wird. Kapokfasern werden heute auf Grund ihres außerordentlich hohen Luftgehaltes von rund 80% und der damit verbundenen Füllkraft und Weichheit als Füllmaterial zum Ausstopfen von Matratzen, Betten, Kissen und/oder Rettungswesten verwendet. Darüber hinaus werden sie infolge ihrer geringen Saugfähigkeit und ihrer hohen Tragkraft zur Füllung von Rettungsgürteln, Schwimmwesten und anderen Rettungsmitteln genutzt.

Die Erfindung hat erkannt, dass aus Kapokfasern durch die Beseitigung ihrer natürlichen Zytotoxizität Fasern gewonnen werden können, welche als ein Ausgangsmaterial für die Herstellung von Hygiene- und/oder Kosmetikprodukten besonders geeignet sind.

Auf Grund ihrer Weichheit bewirken die Kapokfasern eine deutliche Reduktion der durch Hygiene- und/oder Kosmetikprodukte hervorgerufenen mechanischen Irritation beim Benutzer. Dies gilt insbesondere bei solchen Hygiene-und/oder Kosmetikprodukten, welche über einen längeren Zeitraum, beispielsweise einige Stunden, im Kontakt mit der Haut sein können, was beispielsweise bei Stilleinlagen, Tampons, Wundversorgungsprodukten oder Inkontinenzprodukten möglich ist. Aber auch bei solchen Produkten, welche üblicherweise nur kurz in Berührung mit der Haut kommen, beispielsweise Wattepads oder Wattestäbchen zu Reinigungszwecken, erhöht sich bei deren Benutzung der Komfort.

Zytotoxizität der Kapokfasern ist dabei die Fähigkeit der bei Hautkontakt von den Kapokfasern abgegebenen chemischen Substanzen Gewebezellen zu schädigen. Die Zytotoxizität gilt dann als beseitigt, wenn mit einem Verfahren gemäß der Norm DIN EN ISO 10993/5 festgestellt wird, dass die Fasern keine zelltoxischen Substanzen abgeben.

Das erfindungsgemäße Verfahren bewirkt somit, dass chemische Irritationen der Haut des Benutzers durch erfindungsgemäß behandelte Kapokfasern enthaltende Hygiene- und/oder Kosmetikprodukte nicht auftreten, welche die mechanischen Irritation der Haut durch konventionelle Hygiene- und/oder Kosmetikprodukte bei weitem übertreffen könnten.

Durch das erfindungsgemäße Verfahren ist es somit erstmals möglich, das geringe mechanische Irritationspotential der Kapokfasern für Hygiene-und/oder Kosmetikprodukte zu nutzen.

Zudem weisen Kapokfasern eine äußerst geringe Dichte von etwa 0,35 g/cm³ bis 0,90 g/cm³ auf, so dass die daraus gewonnenen Vliese und Hygiene-und/oder Kosmetikprodukte besonders leicht sind, was den Komfort bei deren Anwendung weiter erhöht.

Die so hergestellten Kapokfasern sind zudem biologisch abbaubar. Weiterhin können die als Ausgangsmaterial verwendeten Kapokfasern manuell geerntet und extrahiert werden. Insbesondere können sie ohne Verwendung von Chemikalien, Insektiziden und/oder Pestiziden gewonnen werden, so dass die erfindungsgemäß behandelten Fasern sowie die daraus gewonnenen Vliese und Hygiene- und/oder Kosmetikprodukte besonders umweltfreundlich sind. Zudem sind die erfindungsgemäß behandelten Fasern sowie die daraus gewonnenen Vliese und Hygiene- und/oder Kosmetikprodukte klimafreundlich, da die als Ausgangsmaterial verwendeten Kapokfasern einen nachwachsenden Rohstoff darstellen.

Nach einer vorteilhaften Weiterbildung der Erfindung wird zur Beseitigung der natürliche Zytotoxizität der Kapokfasern ein Bleichverfahren verwendet, welches wenigstens einen Bleichgang zum Entfärben der Kapokfasern umfasst.

Bei Naturfasern ist es allgemein üblich, diese zu Bleichen, um ein ästhetisch ansprechendes Bild der daraus hergestellten Produkte zu erreichen. Der Bleichgang, auch Bleichphase oder Bleichschritt genannt, bildet dabei den Kern des Verfahrens, in dem die eigentliche Entfärbung der Kapokfasern durchgeführt wird. Vor dem Bleichgang können ein oder mehrere Vorbereitungsgänge und nach dem Bleichgang ein oder mehrere Nachbereitungsgänge vorgesehen sein.

Die Erfindung hat nun erkannt, dass es möglich ist, die natürliche Zytotoxizität der Kapokfasern durch ein entsprechend eingerichtetes Bleichverfahren zu beseitigen. Auf diese Weise kann ein einfaches Behandlungsverfahren erreicht werden, da mittels des Bleichverfahrens sowohl die gewünschte Entfärbung als auch die Beseitigung der Zytotoxizität erreicht wird. Das Bleichverfahren kann im grundsätzlichen Aufbau bekannten Verfahren zum Bleichen von Baumwolle entsprechen. Allerdings müssen die Verfahrensparameter des Bleichverfahrens angepasst werden, wenn die Beseitigung der Zytotoxizität erreicht werden soll, da es mittels einer Standardbleiche wie bei Baumwolle nicht gelingt, die natürliche Zytotoxizität der Kapokfaser zu beseitigen.

Nach einer vorteilhaften Weiterbildung der Erfindung wird das Behandlungsverfahren so durchgeführt, dass eine antibakterielle Wirkung der Kapokfasern erhalten bleibt.

Natürliche Kapokfasern enthalten eingelagerte Wirk- und/oder Bitterstoffe, welche eine antibakterielle Wirkung aufweisen. Durch eine entsprechende Wahl der Prozessparameter für das Behandlungsverfahren ist es möglich, die eingelagerten Wirk- und/oder Bitterstoffe zu schonen, so dass die antibakterielle Wirkung auch bei den behandelten Kapokfasern vorliegt. Auf diese Weise können Gesundheitsgefahren bei der Anwendung der aus den derart behandelten Kapokfasern hergestellten Hygiene- und/oder Kosmetikprodukte durch Bakterien vermieden werden.

Eine antibakterielle Wirkung kann dann angenommen werden, wenn diese beispielsweise bei einem Agrarplattendiffusionstest gemäß DIN EN ISO 20645 bei den Testkeimen Staphylococcus Aureus ATCC 6538 (grampositives Bakterium) und/oder Klebsiella Pneumoniae ATCC 4352 (gramnegatives Bakterium festgestellt werden kann.

Nach einer vorteilhaften Weiterbildung der Erfindung wird das Behandlungsverfahren so durchgeführt, dass eine natürliche Wachsschicht der Kapokfasern entfernt wird.

Die unbehandelten Kapokfasern sind mit einer natürlichen Wachsschicht überzogen. Indem nun diese Wachsschicht entfernt wird, kann eine Erhöhung der Reibung zwischen den behandelten Kapokfasern erreicht werden, was die mechanische Stabilität der daraus hergestellten Vliese und der daraus hergestellten Hygiene- und/oder Kosmetikprodukte verbessert.

Zudem kann durch die Entfernung der natürlichen hydrophoben Wachsschicht das Wasseraufnahmevermögen der aus den erfindungsgemäß behandelten Kapokfasern hergestellten Hygiene- und/oder Kosmetikprodukte beträchtlich gesteigert werden. Möglich wird dies insbesondere dadurch, dass so die Kapillarwirkung der hohlen Kapokfasern besonders gut ausgenutzt werden kann. Ebenso kann dadurch die im Vergleich zu Baumwollfasern geringe Kräuselung der Kapokfasern besonders gut ausgenutzt werden. Hierdurch können feste oder pastöse, halbflüssige oder flüssige Medien besser aufgenommen werden, so dass die Hygiene- und/oder Kosmetikprodukte bei gleicher Funktionalität kompakter ausgeführt werden können, was den Anwendungskomfort abermals erhöht.

Gemäß einer vorteilhaften Weiterbildung der Erfindung wird das Behandlungsverfahren so durchgeführt, dass das Wasseraufnahmevermögen der Kapokfasern wenigstens um den Faktor 200, bevorzugt um den Faktor 300, besonders bevorzugt um den Faktor 400 erhöht wird. Die genannten Faktoren können durch eine entsprechend gründliche Entfernung der Wachsschicht erzielt werden. Das Wasseraufnahmevermögen kann dabei gemäß der Norm DIN 53 923 bezogen auf das Eigengewicht der Kapokfasern bei einem daraus hergestellten Wattepad ermittelt werden. Hierdurch ergeben sich bei den daraus hergestellten Hygiene- und/oder Kosmetikprodukte im Vergleich zu aus unbehandelten Kapokfasern hergestellten Hygiene-und/oder Kosmetikprodukte überlegene Eigenschaften. Beispielsweise kann das Wasseraufnahmevermögen bei einem aus unbehandelten Kapokfasern hergestellten Wattepad das 0,1-fache des Eigengewichts und bei einem aus behandelten Kapokfasern hergestellten Wattepad das 40-fache des Eigengewichts betragen.

Gemäß einer vorteilhaften Weiterbildung der Erfindung wird der Bleichgang mittels eines oxidativen Bleichmittels, insbesondere Wasserstoffperoxyd, durchgeführt. Hierdurch kann die Beseitigung der Zytotoxizität in kurzer Zeit und kostengünstig erreicht werden. Grundsätzlich könnten aber auch reduktive Bleichmittel verwendet werden.

Nach einer vorteilhaften Weiterbildung der Erfindung wird der Bleichgang diskontinuierlich in einem Packzylinder durchgeführt. Die Verwendung eines diskontinuierlichen Bleichverfahrens hat sich für die Beseitigung der Zytotoxizität der Kapokfasern als zweckmäßiger als kontinuierliche Verfahren herausgestellt.

Gemäß einer vorteilhaften Weiterbildung der Erfindung beträgt während des Bleichgangs die Dichte der Kapokfasern in dem Packzylinder höchstens 250 g/l, bevorzugt höchstens 200 g/l, und besonders bevorzugt höchstens 150 g/l. Hierdurch wird sichergestellt, dass die Hohlfaserstruktur der Kapokfasern beim Bleichgang nicht mechanisch zerstört wird.

Nach einer vorteilhaften Weiterbildung der Erfindung beträgt während des Bleichgangs die Dichte der Kapokfasern in dem Packzylinder mindestens 50 g/l, bevorzugt mindestens 80 g/l, und besonders bevorzugt mindestens 120 g/l. Hierdurch wird sichergestellt, dass der Bleichgang wirtschaftlich durchgeführt wird.

Nach einer vorteilhaften Weiterbildung der Erfindung wird das Behandlungsverfahren, insbesondere wenigstens der Bleichgang, mit einer maximalen

Prozesstemperatur von höchstens 100 °C, bevorzugt höchstens 90°C, und besonders bevorzugt höchstens 80°C, durchgeführt. Bei diesen vergleichsweise geringen Temperaturen, beim Bleichen von Baumwolle sind Temperaturen von beispielsweise 110°C üblich, kann die Wachsschicht der Kapokfaser noch gut entfernt werden. Darüber hinaus werden die antibakteriellen Wirk- und Bitterstoffe geschont, so dass die antibakterielle Wirkung auch bei den behandelten Kapofasern auftritt. Ebenso wird durch die niederen Temperaturen die Gefahr einer Schädigung der Hohlfaserstruktur verringert.

Nach einer vorteilhaften Weiterbildung der Erfindung wird der Bleichgang bei einer maximalen Prozesstemperatur von mindestens 50 °C, bevorzugt mindestens 60°C, und besonders bevorzugt mindestens 70°C, durchgeführt. Auf diese Weise kann die gewünschte Entfärbung, die gewünschte Entfernung der Wachsschicht sowie die gewünschte Beseitigung der Zytotoxizität in kurzer Zeit erzielt werden.

Nach einer vorteilhaften Weiterbildung der Erfindung beträgt eine Dauer des Bleichgangs wenigstens 2 Stunden, bevorzugt wenigstens 2,5 Stunden, besonders bevorzugt wenigstens 3 Stunden. Bei den angegebenen Werten für die Dauer des Bleichgangs, welche deutlich über den Werten bei herkömmlichen Bleichverfahren für Baumwolle liegen, kann die gewünschte Entfärbung, die gewünschte Entfernung der Wachsschicht sowie die gewünschte Beseitigung der Zytotoxizität auch bei einer oben angegebenen geringen Prozesstemperatur erreicht werden.

Nach einer vorteilhaften Weiterbildung der Erfindung umfasst das Bleichverfahren wenigstens zwei Spülgänge, bevorzugt wenigstens drei Spülgänge, besonders bevorzugt wenigstens vier Spülgänge, zum Entfernen von Chemikalien, welche nach dem Bleichgang durchgeführt werden. Bei einem Spülgang, auch Spülphase oder Spülschritt genannt, werden Chemikalien, beispielsweise das Bleichmittel, Tenside und/oder Bitterstoffe, mittels eines Spülfluids, beispielsweise mit einer Spülflüssigkeit oder mit einem Spülgas, aus dem gebleichten Material entfernt. Indem nun mehrere Spülgänge vorgesehen sind, gelingt dies besonders gut, so dass gelöste aber in dem Kapokfasermaterial verbliebene zytotoxische Stoffe nahezu vollständig entfernt werden können.

Gemäß einer vorteilhaften Weiterbildung der Erfindung umfasst das Bleichverfahren wenigstens einen Netzgang, der vor dem Bleichgang durchgeführt wird. Während des Netzganges, auch Netzphase oder Netzschritt genannt, wird das zu behandelnde Kapokfasermaterial mit Tensiden beaufschlagt. Hierdurch wird das Material besser benetzbar, so dass im nachgeschalteten Bleichgang das Bleichmittel besser zur Wirkung kommt.

Die Aufgabe wird bei Kapokfasern der eingangs genannten Art dadurch gelöst, dass eine natürliche Zytotoxizität der Kapokfasern mittels eines Behandlungsverfahrens, insbesondere mittels eines erfindungsgemäßen Behandlungsverfahrens, beseitigt ist, so dass die Kapokfasern für Hygiene- und/oder Kosmetikprodukte verwendbar sind.

Es ergeben sich die bei dem erfindungsgemäßen Behandlungsverfahren erläuterten Vorteile.

Die Aufgabe wird bei einem Vliesstoff der eingangs genannten Art dadurch gelöst, dass er zytotoxizitätsfreie Kapokfasern umfasst, deren natürliche Zytotoxizität mittels eines Behandlungsverfahrens, insbesondere mittels eines erfindungsgemäßen Behandlungsverfahrens, beseitigt ist.

Bei einem Vliesstoff handelt es sich um ein textiles Flächengebilde aus einzelnen Fasern.

Es ergeben sich die bei dem erfindungsgemäßen Behandlungsverfahren erläuterten Vorteile.

Gemäß einer vorteilhaften Weiterbildung der Erfindung beträgt das Wasseraufnahmevermögen wenigstens das 20-fache, bevorzugt das 30-fache, besonders bevorzugt das 40-fache, des Eigengewichts. Im Vergleich zu reinen Vliesstoffen aus Baumwolle, bei denen das Wasseraufnahmevermögen typischerweise das 12- bis 15-fache des Eigengewichts beträgt, können aus einem derartigen Vliesstoff Hygiene- und/oder Kosmetikprodukte mit überlegenen Eigenschaften hinsichtlich ihrer Kompaktheit bei einer vorgesehenen absoluten Wasseraufnahmefähigkeit, welche beispielsweise in Gramm oder Milliliter angegeben werden kann, hergestellt werden.

Nach einer vorteilhaften Weiterbildung der Erfindung umfasst der Vliesstoff eine Mischung mehrerer Faserarten, welche neben zytotoxizitätsfreien Kapokfasern beispielsweise Baumwollfasern und/oder Viskosefasern enthält. Auf diese Weise können die Vorteile unterschiedlicher Faserarten gezielt kombiniert werden. So kann beispielsweise durch einen Anteil von zytotoxizitätsfreien Kapokfasern das Wasseraufnahmevermögen erhöht und durch einen Anteil von Baumwollfasern und/oder Viskosefasern auf Grund ihrer höheren Reibwerte die mechanische Stabilität des Vliesstoffes verbessert werden.

Nach einer vorteilhaften Weiterbildung der Erfindung beträgt der Anteil der zytotoxizitätsfreien Kapokfasern an der Mischung wenigstens 3%, bevorzugt wenigstens 5%, besonders bevorzugt wenigstens 10%. Bereits durch einen derart geringen Anteil an zytotoxizitätsfreien Kapokfasern kann das Wasseraufnahmevermögen des Vliesstoffes deutlich erhöht werden, wobei keine nennenswerte Beeinträchtigung der mechanischen Stabilität eintritt.

Nach einer vorteilhaften Weiterbildung der Erfindung beträgt der Anteil der zytotoxizitätsfreien Kapokfasern an der Mischung höchstens 70%, bevorzugt höchstens 60%, besonders bevorzugt höchstens 50%. Bei diesen Höchstwerten kann eine hinreichende mechanische Stabilität des Vliesstoffes noch gewährleistet werden und gleichzeitig das Wasseraufnahmevermögen des Vliesstoffes optimiert werden.

Gemäß einer vorteilhaften Weiterbildung der Erfindung umfasst der Vliesstoff Hilfsstoffe, insbesondere Weichmacher, Füllstoffe und/oder Bindemittel. Auf diese Weise können insbesondere die mechanischen Eigenschaften des Vliesstoffes und der daraus hergestellten Kosmetik- und/oder Hygieneprodukte gezielt beeinflusst werden.

Nach einer vorteilhaften Weiterbildung der Erfindung beträgt das Flächengewicht höchstens 300 g/m², bevorzugt höchstens 250 g/m², besonders bevorzugt höchstens 200 g/m². Vliesstoffe mit einem derartigen Mindestflächengewicht sind zur Herstellung von Kosmetik- und/oder Hygieneprodukte besonders gut geeignet.

Gemäß einer vorteilhaften Weiterbildung der Erfindung beträgt das Flächengewicht mindestens 50 g/m², bevorzugt mindestens 100 g/m², besonders bevorzugt mindestens 150 g/m². Vliesstoffe mit einem derartigen Höchstflächengewicht sind zur Herstellung von Kosmetik- und/oder Hygieneprodukte besonders gut geeignet.

Die Aufgabe wird bei einem Hygiene- und/oder Kosmetikprodukt der eingangs genannten Art dadurch gelöst, dass der Vliesstoff erfindungsgemäß ausgebildet ist.

Es ergeben sich die bei dem erfindungsgemäßen Vliesstoff erläuterten Vorteile.

Die vorstehend erläuterten und/oder in den Unteransprüchen wiedergegebenen vorteilhaften Aus- und Weiterbildungen der Erfindung können dabei - außer z. B. in den Fällen eindeutiger Abhängigkeiten oder unvereinbarer Alternativen - einzeln oder aber auch in beliebiger Kombination miteinander zur Anwendung kommen.

Die Erfindung und ihre vorteilhaften Aus- und Weiterbildungen sowie deren Vorteile werden nachfolgend anhand von Zeichnungen näher erläutert. Es zeigen, jeweils in einer schematischen Prinzipskizze:
- **Figur 1**: eine schematisierte räumliche Darstellung eines Ausführungsbeispiels eines erfindungsgemäßen Wattepads als Beispiel für ein erfindungsgemäßes Hygiene- und/oder Kosmetikprodukt und
- **Figur 2**: ein Bleichverfahren, welches Teil eines Ausführungsbeispiels eines erfindungsgemäßen Behandlungsverfahrens ist, in einer Blockdarstellung.

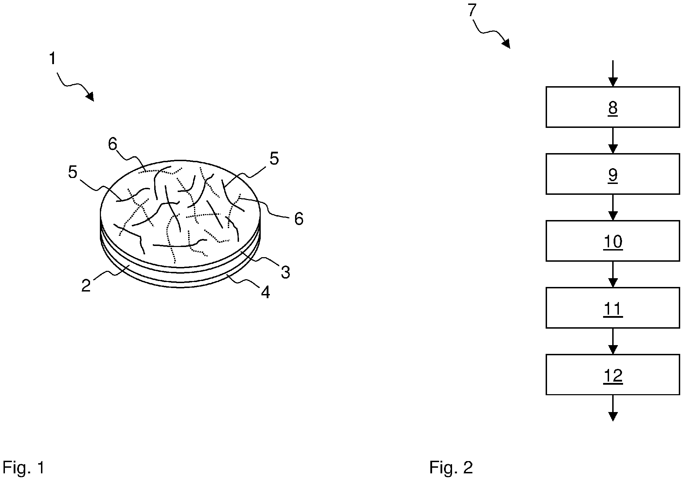

Figur 1 zeigt en Ausführungsbeispiel eines erfindungsgemäßen Wattepads 1 als Beispiel für ein erfindungsgemäßes Hygiene- und/oder Kosmetikprodukt 1, welches beispielsweise zum Reinigen der Haut des Benutzers vorgesehen ist. Das Wattepad 1 umfasst einen ersten Vliesstoff 2, einen zweiten Vliesstoff 3 sowie einen dritten Vliesstoff 4, welche schichtartig angeordnet und dauerhaft verbunden sind. Der erste Vliesstoff 2 bildet dabei eine Saugschicht 2, während der zweite Vliesstoff 3 eine obere Deckschicht 3 und der dritte Vliesstoff 4 eine untere Deckschicht 4 bildet.

Die Vliesstoffe 2, 3, 4 bestehen jeweils aus einer Mischung von erfindungsgemäßen Kapokfasern 5 und von Baumwollfasern 6, von welchen jeweils nur einige symbolisch dargestellt sind.

Auf Grund ihrer Weichheit bewirken die Kapokfasern 5 eine deutliche Reduktion der durch das Wattepad 1 hervorgerufenen mechanischen Irritation beim Benutzer. Hierdurch erhöht sich bei dessen Benutzung der Komfort.

Die Kapokfasern 5 zeichnen sich dadurch aus, dass die natürliche Zytotoxizität der Kapokfasern mittels eines Behandlungsverfahrens beseitigt ist. Bei dem erfindungsgemäßen Wattepad 1 ist somit verhindert, dass chemische Irritationen der Haut des Benutzers auftreten, welche die mechanischen Irritationen der Haut durch konventionelle Hygiene- und/oder Kosmetikprodukte bei weitem übertreffen könnten.

Zudem weisen die Kapokfasern 5 eine äußerst geringe Dichte von etwa 0,35 g/cm³ bis 0,90 g/cm³ auf, so dass die daraus gewonnenen Vliese 2, 3 4 und das Wattepad 1 besonders leicht sind, was den Komfort bei dessen Anwendung weiter erhöht.

Die verwendeten Kapokfasern 5 sind zudem biologisch abbaubar. Weiterhin können die als Ausgangsmaterial verwendeten Kapokfasern manuell geerntet und extrahiert werden. Insbesondere können sie. ohne Verwendung von Chemikalien, Insektiziden und/oder Pestiziden gewonnen werden, so dass die erfindungsgemäß behandelten Fasern sowie die daraus gewonnenen Vliese und das Wattepad 1 besonders umweltfreundlich sind. Zudem sind die verwendeten Kapokfasern 5 sowie die daraus gewonnenen Vliese 2, 3, 4 und das Wattepad 1 klimafreundlich, da die als Ausgangsmaterial verwendeten Kapokfasern einen nachwachsenden Rohstoff darstellen.

Der Anteil der zytotoxizitätsfreien Kapokfasern 5 an der jeweiligen Mischung der Vliesstoffe 2, 3, 4 kann wenigstens 3%, bevorzugt wenigstens 5%, besonders bevorzugt wenigstens 10% und höchstens 70%, bevorzugt höchstens 60%, besonders bevorzugt höchstens 50%, betragen. Dabei kann die Mischung des zweiten Vliesstoffes 3 deutlich mehr Kapokfasern 5 enthalten als die Mischung des zweiten Vliesstoffes 3 und des dritten Vliesstoffes 4, um die Saugfähigkeit der durch ihn gebildeten Saugschicht 2 zu optimieren. Gleichzeitig wird so die mechanische Stabilität des zweiten Vliesstoffes 3 und des dritten Vliesstoffes 4, welche jeweils eine mechanisch stärker beanspruchte Deckschicht 3, 4 bilden, an die Erfordernisse angepasst. Beispielsweise können die Mischungen des zweiten Vliesstoffes 3 und des dritten Vliesstoffes 4 20% Kapokfasern und 80% Baumwollfasern enthalten, womit ein Wasseraufnahmevermögen vom 23-fachen des Eigengewichts einhergehen kann. Ebenso kann die Mischung des ersten Vliesstoffes 2 50% Kapokfasern und 50% Baumwollfasern enthalten, womit ein Wasseraufnahmevermögen vom 30-fachen des Eigengewichts einhergehen kann.

Die Vliesstoffe 2, 3, 4 können Hilfsstoffe, insbesondere Weichmacher, Füllstoffe und/oder Bindemittel umfassen. Auf diese Weise können insbesondere die mechanischen Eigenschaften des Vliesstoffes und der daraus hergestellten Kosmetik- und/oder Hygieneprodukte gezielt beeinflusst werden

Dabei können die Vliesstoffe 2, 3, 4 ein Flächengewicht von höchstens 300 g/m², bevorzugt höchstens 250 g/m², besonders bevorzugt höchstens 200 g/m² und von mindestens 50 g/m², bevorzugt mindestens 100 g/m², besonders bevorzugt mindestens 150 g/m², aufweisen. Zweckmäßigerweise weisen die als Deckschichten 3, 4 dienenden zweiten und dritten Vliesstoffe 3, 4 ein geringeres Flächengewicht als der als Saugschicht 2 dienende erste Vliesstoff 2 auf.

Figur 2 zeigt ein Bleichverfahren 7, welches Teil eines Ausführungsbeispiels eines erfindungsgemäßen Behandlungsverfahrens ist, in einer Blockdarstellung. Das Bleichverfahren 7 umfasst n dieser Reihenfolge einen Netzgang 8, einen Bleichgang 9, einen ersten Spülgang 10, einen zweiten Spülgang 11 und einen dritten Spülgang 12. Das erfindungsgemäße Behandlungsverfahren kann vor der Durchführung des Bleichverfahrens 7 vorsehen, dass die Kapokfasern in herkömmlicher Weise mechanisch geöffnet, gereinigt und entstaubt werden. Ebenso kann das erfindungsgemäße Behandlungsverfahren nach der Durchführung des Bleichverfahrens 7 vorsehen, dass die Kapokfasern 5 mit Weichmachern oder anderen Chemikalien behandelt werden. Nach der Durchführung des erfindungsgemäßen Behandlungsverfahrens können die behandelten Kapokfasern 5 in herkömmlicher Weise zu einem Vlies 2, 3, 4 oder zu einem Garn verarbeitet werden.

Das erfindungsgemäße Behandlungsverfahren ist so gestaltet, dass eine natürliche Zytotoxizität der Kapokfasern 5 beseitigt wird, so dass die Kapokfasern 5 für Hygiene- und/oder Kosmetikprodukte 1 verwendbar sind. Die Beseitigung der natürlichen Zytotoxizität der Kapokfasern 5 wird dabei im Wesentlichen durch das Bleichverfahren 7 bewirkt.

Die Erfindung hat nun erkannt, dass es möglich ist, die natürliche Zytotoxizität der Kapokfasern 5 durch ein entsprechend eingerichtetes Bleichverfahren 7 zu beseitigen. Auf diese Weise kann ein einfaches Behandlungsverfahren erreicht werden, da mittels des Bleichverfahrens 7 sowohl die gewünschte Entfärbung als auch die Beseitigung der Zytotoxizität erreicht wird.

Dabei kann das Behandlungsverfahren insgesamt so durchgeführt werden, dass eine antibakterielle Wirkung der Kapokfasern 5 erhalten bleibt.

Das Behandlungsverfahren kann insgesamt so durchgeführt werden, dass eine natürliche Wachsschicht der Kapokfasern 5 entfernt wird.

Die unbehandelten Kapokfasern sind mit einer natürlichen Wachsschicht überzogen. Indem nun diese Wachsschicht entfernt wird, kann eine Erhöhung der Reibung zwischen den behandelten Kapokfasern 5 erreicht werden, was die mechanische Stabilität der daraus hergestellten Vliese 2, 3, 4 und der daraus hergestellten Hygiene- und/oder Kosmetikprodukte 1 verbessert.

Zudem kann durch die Entfernung der natürlichen hydrophoben Wachsschicht das Wasseraufnahmevermögen der aus den erfindungsgemäß behandelten Kapokfasern 5 hergestellten Hygiene- und/oder Kosmetikprodukte 1 beträchtlich gesteigert werden. Hierdurch können feste oder pastöse, halbflüssige oder flüssige Medien besser aufgenommen werden, so dass die Hygiene- und/oder Kosmetikprodukte 1 bei gleicher Funktionalität kompakter ausgeführt werden können, was den Anwendungskomfort abermals erhöht.

Zweckmäßigerweise wird das Behandlungsverfahren so durchgeführt, dass das Wasseraufnahmevermögen der Kapokfasern 5 bzw. der daraus hergestellten Kosmetik- und/oder Hygieneprodukte 1 wenigstens um den Faktor 200, bevorzugt um den Faktor 300, besonders bevorzugt um den Faktor 400 erhöht wird. Hierdurch ergeben sich bei den daraus hergestellten Hygiene-und/oder Kosmetikprodukte 1 im Vergleich zu aus unbehandelten Kapokfasern hergestellten Hygiene- und/oder Kosmetikprodukte überlegene Eigenschaften.

Während des Netzganges 8, auch Netzphase oder Netzschritt genannt, wird das zu behandelnde Kapokfasermaterial mit Tensiden beaufschlagt. Hierdurch wird das Material besser benetzbar, so dass im nachgeschalteten Bleichgang 9 das Bleichmittel besser zur Wirkung kommt.

Der Bleichgang 9 kann mittels eines oxidativen Bleichmittels, insbesondere Wasserstoffperoxyd, durchgeführt werden. Hierdurch kann die Beseitigung der Zytotoxizität in kurzer Zeit und kostengünstig erreicht werden. Grundsätzlich könnten aber auch reduktive Bleichmittel verwendet werden.

Zweckmäßigerweise wird der Bleichgang 9 diskontinuierlich in einem Packzylinder durchgeführt. Die Verwendung eines diskontinuierlichen Bleichverfahrens 7 hat sich für die Beseitigung der Zytotoxizität der Kapokfasern als zweckmäßiger als kontinuierliche Verfahren herausgestellt.

Bevorzugt beträgt während des Bleichgangs 9 die Dichte der Kapokfasern in dem Packzylinder höchstens 250 g/l, bevorzugt höchstens 200 g/l, und besonders bevorzugt höchstens 150 g/l. Hierdurch wird sichergestellt, dass die Hohlfaserstruktur der Kapokfasern 5 beim Bleichgang 9 nicht mechanisch zerstört wird.

Dabei beträgt zweckmäßigerweise während des Bleichgangs 9 die Dichte der Kapokfasern 5 in dem Packzylinder mindestens 50 g/l, bevorzugt mindestens 80 g/l, und besonders bevorzugt mindestens 120 g/l. Hierdurch wird sichergestellt, dass der Bleichgang wirtschaftlich durchgeführt wird.

Das Behandlungsverfahren kann vorsehen, dass es, insbesondere wenigstens der Bleichgang 9, mit einer maximalen Prozesstemperatur von höchstens 100 °C, bevorzugt höchstens 90°C, und besonders bevorzugt höchstens 80°C, durchgeführt wird. Bei diesen vergleichsweise geringen Temperaturen, beim Bleichen von Baumwolle sind Temperaturen von beispielsweise 110°C üblich, kann die Wachsschicht der Kapokfaser 5 noch gut entfernt werden. Darüber hinaus werden die antibakteriellen Wirk- und Bitterstoffe geschont, so dass die antibakterielle Wirkung auch bei den behandelten Kapofasern 5 auftritt. Ebenso wird durch die niederen Temperaturen die Gefahr einer Schädigung der Hohlfaserstruktur verringert.

Zweckmäßigerweise wird der Bleichgang 9 bei einer maximalen Prozesstemperatur von mindestens 50 °C, bevorzugt mindestens 60°C, und besonders bevorzugt mindestens 70°C, durchgeführt. Auf diese Weise kann die gewünschte Entfärbung, die gewünschte Entfernung der Wachsschicht sowie die gewünschte Beseitigung der Zytotoxizität in kurzer Zeit erzielt werden.

Dabei kann die Dauer des Bleichgangs 9 wenigstens 2 Stunden, bevorzugt wenigstens 2,5 Stunden, besonders bevorzugt wenigstens 3 Stunden, betragen. Bei den angegebenen Werten für die Dauer des Bleichgangs 9, welche deutlich über den Werten bei herkömmlichen Bleichverfahren für Baumwolle liegen, kann die gewünschte Entfärbung, die gewünschte Entfernung der Wachsschicht sowie die gewünschte Beseitigung der Zytotoxizität auch bei einer oben angegebenen geringen Prozesstemperatur erreicht werden.

Im Ausführungsbeispiel umfasst das Bleichverfahren 7 drei Spülgänge 10, 11, 12 zum Entfernen von Chemikalien, welche nach dem Bleichgang 9 durchgeführt werden. Bei jedem der Spülgänge 10, 11, 12, auch Spülphase oder Spülschritt genannt, werden Chemikalien, beispielsweise das Bleichmittel, Tenside und/oder Bitterstoffe, mittels eines Spülfluids, beispielsweise mit einer Spülflüssigkeit oder mit einem Spülgas, aus dem gebleichten Material entfernt. Indem nun mehrere Spülgänge 10, 11, 12 vorgesehen sind, gelingt dies besonders gut, so dass gelöste aber in dem Kapokfasermaterial verbliebene zytotoxische Stoffe nahezu vollständig entfernt werden können.

### Bezugszeichenliste

- 1: Hygiene- und/oder Kosmetikprodukt, Wattepad
- 2: erster Vliesstoff, Saugschicht
- 3: zweiter Vliesstoff, obere Deckschicht
- 4: dritter Vliesstoff, untere Deckschicht
- 5: Kapokfasern
- 6: Baumwollfasern
- 7: Bleichverfahren
- 8: Netzgang
- 9: Bleichgang
- 10: erster Spülgang
- 11: zweiter Spülgang
- 12: dritter Spülgang

## Patentansprüche

1. Behandlungsverfahren für Kapokfasern (5), **dadurch gekennzeichnet, dass** eine natürliche Zytotoxizität der Kapokfasern (5) beseitigt wird, so dass die Kapokfasern für Hygiene- und/oder Kosmetikprodukte verwendbar sind, wobei vorzugsweise zur Beseitigung der natürlichen Zytotoxizität der Kapokfasern (5) ein Bleichverfahren (7) verwendet wird, welches wenigstens einen Bleichgang (9) zum Entfärben der Kapokfasern (5) umfasst.

2. Behandlungsverfahren nach einem der vorstehenden Ansprüche, **dadurch gekennzeichnet, dass** es so durchgeführt wird, dass eine natürliche antibakterielle Wirkung der Kapokfasern (5) erhalten bleibt und/oder dass es so durchgeführt wird, dass eine natürliche Wachsschicht der Kapokfasern (5) im Wesentlichen entfernt wird.

3. Behandlungsverfahren nach einem der vorstehenden Ansprüche, **dadurch gekennzeichnet, dass** es so durchgeführt wird, dass das Wasseraufnahmevermögen der Kapokfasern (5) wenigstens um den Faktor 200, bevorzugt um den Faktor 300, besonders bevorzugt um den Faktor 400 erhöht wird.

4. Behandlungsverfahren nach einem der vorstehenden Ansprüche, **dadurch gekennzeichnet, dass** der Bleichgang (9) mittels eines oxidativen Bleichmittels, insbesondere Wasserstoffperoxyd, durchgeführt wird und/oder dass eine Dauer des Bleichgangs (9) wenigstens 2 Stunden, bevorzugt wenigstens 2,5 Stunden, besonders bevorzugt wenigstens 3 Stunden beträgt.

5. Behandlungsverfahren nach einem der vorstehenden Ansprüche, **dadurch gekennzeichnet, dass** der Bleichgang (9) diskontinuierlich in einem Packzylinder durchgeführt wird.

6. Behandlungsverfahren nach einem der vorstehenden Ansprüche, **dadurch gekennzeichnet, dass** während des Bleichgangs (9) die Dichte der Kapokfasern (5) in dem Packzylinder höchstens 250 g/l, bevorzugt höchstens 200 g/l, und besonders bevorzugt höchstens 150 g/l, beträgt und/oder dass während des Bleichgangs (9) die Dichte der Kapokfasern (5) in dem Packzylinder mindestens 50 g/l, bevorzugt mindestens 80 g/l, und besonders bevorzugt mindestens 120 g/l, beträgt.

7. Behandlungsverfahren nach einem der vorstehenden Ansprüche, **dadurch gekennzeichnet, dass** das Behandlungsverfahren, insbesondere wenigstens der Bleichgang (9), mit einer maximalen Prozesstemperatur von höchstens 100 °C, bevorzugt höchstens 90°C g/l, und besonders bevorzugt höchstens 80°C, durchgeführt wird und/oder dass der Bleichgang (9) bei einer maximalen Prozesstemperatur von mindestens 50 °C, bevorzugt mindestens 60°C, und besonders bevorzugt mindestens 70°C, durchgeführt wird.

8. Behandlungsverfahren nach einem der vorstehenden Ansprüche, **dadurch gekennzeichnet, dass** das Bleichverfahren (7) wenigstens zwei Spülgänge (10, 11, 12), bevorzugt wenigstens drei Spülgänge (10, 11, 12), besonders bevorzugt wenigstens vier Spülgänge (10, 11, 12), zum Entfernen von Chemikalien umfasst, welche nach dem Bleichgang (9) durchgeführt werden und/oder dass das Bleichverfahren (7) wenigstens einen Netzgang (8) umfasst, der vor dem Bleichgang (9) durchgeführt wird.

9. Kapokfasern, **dadurch gekennzeichnet, dass** eine natürliche Zytotoxizität der Kapokfasern (5) mittels eines Behandlungsverfahrens, insbesondere mittels eines Behandlungsverfahrens nach einem der vorstehenden Ansprüche, beseitigt ist, so dass die Kapokfasern für Hygiene-und/oder Kosmetikprodukte verwendbar sind.

10. Vliesstoff für Hygiene- und/oder Kosmetikprodukte, **dadurch gekennzeichnet, dass** er zytotoxizitätsfreie Kapokfasern (5) umfasst, deren natürliche Zytotoxizität mittels eines Behandlungsverfahrens, insbesondere mittels eines Behandlungsverfahrens nach einem der Ansprüche 1 bis 8, beseitigt ist.

11. Vliesstoff nach Anspruch 10, **dadurch gekennzeichnet, dass** das Wasseraufnahmevermögen wenigstens das 20-fache, bevorzugt das 30-fache, besonders bevorzugt das 40-fache, des Eigengewichts beträgt.

12. Vliesstoff nach Anspruch 10 oder 11, **dadurch gekennzeichnet, dass** er eine Mischung mehrerer Faserarten umfasst, welche neben zytotoxizitätsfreien Kapokfasern (5) beispielsweise Baumwollfasern (6) und/oder Viskosefasern enthält und/oder dass er Hilfsstoffe, insbesondere Weichmacher, Füllstoffe und/oder Bindemittel, umfasst.

13. Vliesstoff nach Anspruch 12, **dadurch gekennzeichnet, dass** der Anteil der zytotoxizitätsfreien Kapokfasern (5) an der Mischung wenigstens 3%, bevorzugt wenigstens 5%, besonders bevorzugt wenigstens 10%, beträgt und/oder dass der Anteil der zytotoxizitätsfreien Kapokfasern (5) an der Mischung höchstens 70%, bevorzugt höchstens 60%, besonders bevorzugt höchstens 50%, beträgt.

14. Vliesstoff nach einem der Ansprüche 10 bis 13, **dadurch gekennzeichnet, dass** das Flächengewicht höchstens 300 g/m², bevorzugt höchstens 250 g/m², besonders bevorzugt höchstens 200 g/m², beträgt und/oder dass das Flächengewicht mindestens 50 g/m², bevorzugt mindestens 100 g/m², besonders bevorzugt mindestens 150 g/m², beträgt.

15. Hygiene- und/oder Kosmetikprodukt, insbesondere Wattepad (1), Stilleinlage, Tampon, Wattestäbchen, Wundversorgungsprodukt oder Inkontinenzprodukt, umfassend wenigstens einen Vliesstoff (2, 3, 4), **dadurch gekennzeichnet, dass** der Vliesstoff (2, 3, 4) nach einem der Ansprüche 10 bis 13 ausgebildet ist.
